# EUROPEAN PATENT APPLICATION

(11) **EP 4 684 738 A1**
(43) Date of publication of application: **28.01.2026**
(21) Application number: 25191809.0
(22) Date of filing: 25.07.2025
(51) Int. Cl.: A61B 8/08, A61B 17/34

(54) **GUIDING SYSTEM FOR ULTRASONIC TRANSDUCER**

(30) Priority: 25.07.2024 US 202463675363 P
(71) Applicant: Sononurse Vs Inc., Verdun QC H3E 0C8 (CA)
(72) Inventor: ZINE, Yahia, Verdun, H3E 0C8 (CA); POULIN, Samuel, Verdun, H3E 0C8 (CA); VUCH, Damian, Verdun, H3E 0C8 (CA); CHERY, Eliott, Verdun, H3E 0C8 (CA)
(74) Representative: Regimbeau

(57) **Abstract**

A guiding system for an ultrasonic transducer having a body, has: a reference member releasably secured to the body and having an attachment member defining a reference face; a guiding member having: a sliding section defining a sliding reference face, and a guiding section connected to the sliding section, the guiding section configured to hold a surgical tool; and a magnetic connection to magnetically connect the sliding section of the guiding member to the attachment member and to maintain the reference face of the attachment member in contact with the sliding reference face while allowing translation and rotation of the guiding member relative to the attachment member, the magnetic connection created by a magnet mounted to one of the attachment member and the sliding section of the guiding member and a magnetically-attractable member mounted to the other of the attachment member and the sliding section of the guiding member.

## Description

### CROSS-REFERENCES TO RELATED APPLICATIONS

The application claims benefit from United States provisional application no. 63/675,363 filed on July 25, 2024, the entire contents of which are incorporated by reference herein.

### TECHNICAL FIELD

The application relates generally to medical devices and, more particularly, to systems and methods used for guiding a surgical tool, such as a needle or catheter, during insertion in a patient using an imaging system, such as an ultrasound transducer.

### BACKGROUND

An ultrasound transducer is a medical device that emits and receives sound waves for visualizing internal structures of a body. In some applications, these transducers are used to help a medical practitioner insert a needle or catheter into a blood vessel (e.g., vein, artery) of a patient, nerves, or any target in the body (e.g., biopsy for tumor, infiltration in junctions, mini-invasive surgery). The medical practitioner may align the needle within a scanning plane of the transducer to visualise the needle as it is being inserted in the patient. Typically, these operations are free-handed and rely on dexterity and experience of the medical practitioner. Although satisfactory to some extent, there always remains a need for improvement.

### SUMMARY

In one aspect, there is provided a guiding system for an ultrasonic transducer having a body, comprising: a reference member releasably secured to the body of the ultrasonic transducer, the reference member having an attachment member defining a reference face; a guiding member having: a sliding section defining a sliding reference face, and a guiding section connected to the sliding section, the guiding section configured to hold a surgical tool for insertion into a patient; and a magnetic connection to magnetically connect the sliding section of the guiding member to the attachment member and to maintain the reference face of the attachment member in contact with the sliding reference face of the sliding section while allowing translation and rotation of the guiding member relative to the attachment member, the magnetic connection created by a magnet mounted to one of the attachment member and the sliding section of the guiding member and a magnetically-attractable member mounted to the other of the attachment member and the sliding section of the guiding member.

The guiding system described above may include any of the following features, in any combinations.

In some embodiments, the reference face is circular to facilitate rotation of the guiding member relative to the reference member.

In some embodiments, the reference member includes a collar mounted at least partially around the body of the ultrasonic transducer, the attachment member mounted to the collar.

In some embodiments, the attachment member includes three attachment members disposed around the body of the ultrasonic transducer, the three attachment members spaced apart from one another by 90 degrees.

In some embodiments, the three attachment members include two in-plane attachment members being located on opposite sides of the body of the ultrasonic transducer, the two in-plane attachment members located on a long side of the body.

In some embodiments, one or more of: reference faces of the two in-plane attachment members face directions being transversal to a scanning plane of the ultrasonic transducer; and the three attachment members include an out-of-plane attachment member disposed circumferentially between the two in-plane attachment members, the out-of-plane attachment member facing a direction being parallel to a scanning plane of the ultrasonic transducer.

In some embodiments, the attachment member includes a magnet and wherein the sliding section includes a magnetically-attractable plate defining the sliding reference face.

In some embodiments, the magnetically-attractable plate includes a second magnet configured to attract the magnet of the attachment member.

In some embodiments, the sliding section defines rails, the sliding reference face located between the rails, the rails configured to maintain the reference face of the attachment member in contact with the sliding reference face.

In some embodiments, the guiding system for an ultrasonic transducer having a body includes a holding mechanism engageable to the guiding section for holding the surgical tool, the holding mechanism having: a connecting member pivotably engaged to the guiding section; and a holder detachably engageable to the connecting member, the holder configured to support the surgical tool.

In some embodiments, the connecting member includes a longitudinal arm configured to be received between fingers of a user and a handle configured to be engaged by a hand of the user, the longitudinal arm protruding transversally from the handle, the connecting member having a support member secured to the handle, the holder detachably secured to the support member.

In some embodiments, the holder is configured to be disconnected from the connecting member while complying with an Aseptic Non Touch Technique (ANTT), and wherein the guiding system is further configured to maintain the catheter in a stable position relative to the insertion site during disconnection.

In some embodiments, the disconnection is assisted by an auxiliary component configured to aid in the separation of the holder and the connecting member while complying with an Aseptic Non Touch Technique (ANTT).

In another aspect, there is provided an ultrasonic transducer, comprising: a body having a sensing end configured to emit ultrasound in a sensing plane; and a guiding system secured to the body, the guiding system having: an attachment member defining a reference face; a guiding member having: a sliding section defining a sliding reference face, and a guiding section connected to the sliding section, the guiding section configured to hold a surgical tool for insertion into a patient; and a magnetic connection to magnetically connect the sliding section of the guiding member to the attachment member and to maintain the reference face of the attachment member in contact with the sliding reference face of the sliding section while allowing translation and rotation of the guiding member relative to the attachment member, the magnetic connection defined by a magnet mounted to one of the attachment member and the sliding section of the guiding member and a magnetically-attractable member mounted to the other of the attachment member and the sliding section of the guiding member.

The ultrasonic transducer described above may include any of the following features, in any combinations.

In some embodiments, the reference face is circular to facilitate rotation of the guiding member relative to the reference face.

In some embodiments, the attachment member includes three attachment members disposed around the body of the ultrasonic transducer, the three attachment members spaced apart from one another by 90 degrees.

In some embodiments, the three attachment members include two in-plane attachment members being located on opposite sides of the body of the ultrasonic transducer, the two in-plane attachment members located on a long side of the body.

In some embodiments, one or more of: reference faces of the two in-plane attachment members face directions being transversal to a scanning plane of the ultrasonic transducer; the three attachment members include an out-of-plane attachment member disposed circumferentially between the two in-plane attachment members, the out-of-plane attachment member facing a direction being parallel to a scanning plane of the ultrasonic transducer.

In some embodiments, the attachment member includes a magnet and wherein the sliding section includes a magnetically-attractable plate defining the sliding reference face.

In some embodiments, the ultrasonic transducer includes a longitudinal magnet secured to the sliding section and configured to attract the magnet of the attachment member.

In some embodiments, the sliding section defines rails, the sliding reference face located between the rails, the rails configured to maintain the reference face of the attachment member in contact with the sliding reference face.

In yet another aspect, there is provided a method of guiding a surgical tool using an ultrasonic transducer, comprising: positioning the ultrasonic transducer relative to a patient until a scanning plane of the ultrasonic transducer intersects a blood vessel of the patient; magnetically connecting a guiding member to an attachment member, the attachment member being mounted to the ultrasonic transducer; and engaging the surgical tool to the guiding member.

### BRIEF DESCRIPTION OF THE DRAWINGS

Reference is now made to the accompanying figures in which:
Fig. 1 is a three dimensional exploded view of a guiding system to be coupled to an ultrasonic transducer;
Fig. 2 is a three dimensional view of the guiding system;
Figs. 3A and 3B are three dimensional views of the guiding system shown in different positions one relative to the other;
Fig. 4 is a rear three dimensional view of a guiding member of the guiding system;
Figs. 5A to 5C are three dimensional views illustrating steps of assembling a reference member of the guiding system to a cover of the guiding system;
Fig. 6 is a top three dimensional view of the cover and of a rotating member secured thereto;
Figs. 7A and 7B are three dimensional exploded views illustrating steps of assembling the rotating member to the cover of the guiding system;
Figs. 8A to 8C are three dimensional views of the rotating member shown in different positions relative to the cover;
Fig. 9 is a three dimensional view of a guiding member in accordance with another embodiment and including a catheter engaged thereto via a holding mechanism including a holder and a connecting member;
Fig. 10 is a three dimensional view of the guiding member of Fig. 9;
Fig. 11 is a three dimensional exploded view of the catheter and holding mechanism;
Fig. 12 is a three dimensional view of a connecting member of the holding mechanism of Fig. 11;
Fig. 13 is a three dimensional view of a holder of the holding mechanism of Fig. 11;
Fig. 14 is a three dimensional view of the catheter engaged to the holding mechanism;
Fig. 15 is a three dimensional view of a connecting member in accordance with another embodiment to be used with the guiding member of Fig. 10;
Fig. 16 is a top view of the connecting member of Fig. 15 shown engaged by a left hand of a practitioner; and
Fig. 17 is a flowchart illustrating steps of a method of guiding a surgical tool, such as a needle or a catheter using an ultrasonic transducer.

### DETAILED DESCRIPTION

As explained above, in the context of needle manipulation as a function of ultrasound tracking, medical practitioners need skills and experience to appropriately position a needle within the necessary plane. Indeed, the practitioner may easily lose track of the needle in the output image upon a slight movement of the transducer as the ultrasound beam is thin. Understandably, existing techniques require a high level of expertise as well as excellent hand-eye coordination, and only capable of being performed by highly trained specialists.

In anesthesiology, ultrasound may be used to guide the placement of needles when injecting local anesthetic solutions in the proximity of nerves identified within an ultrasound image. Ultrasound is also used for vascular access such as cannulation of large central veins and for arterial cannulation. Ultrasound transducers provide medical practitioners a view of a single scanning plane - typically including a cross-sectional or longitudinal view of the needle. However, neither of these single scanning planes provides all the information necessary to perform such medical procedures. For example, the ultrasound guided application of an anesthetic requires the placement of a needle in close proximity of a nerve without penetrating it.

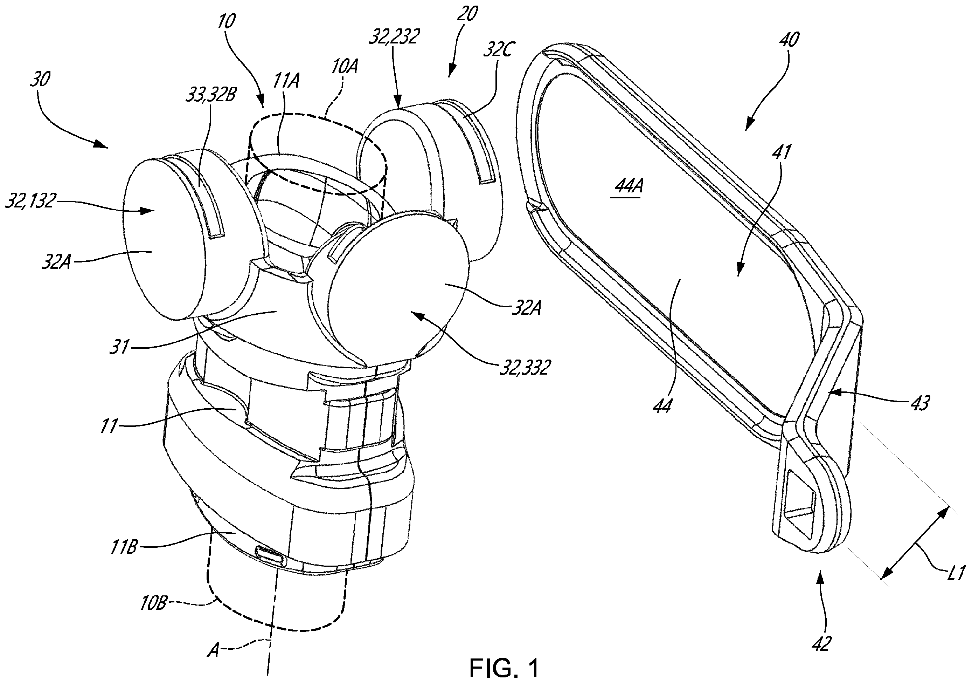

Referring now to Fig. 1, an ultrasonic transducer, referred to below as "transducer" is shown schematically at 10 equipped with a guiding system at 20. The transducer 10 is configured to provide imaging of an inner portion of a body of a patient. The transducer 10 includes a body extending from a connecting end 10A to a sensing end 10B. The connecting end 10A is the end via which the transducer 10 is operatively connected to a device used to visually display the inner portion of the body of the patient. The sensing end 10B is the end that contacts the patient and via which the ultrasonic waves are emitted and sensed to provide the visual images of the patient. The sending end 10B emits and receives ultrasound on a scanning plane. The expression "sensing plane" implies that the ultrasounds are emitted parallel to or along that plane, though this is optional as other arrangements are possible. The sensing plane therefore intersects the body of the patient and what the practitioner sees corresponds to the parts of the body that have been intersected by this plane.

The transducer 10 may be equipped with the guiding system 20, which includes a cover 11, which may be referred to as a housing, an enclosure, and so on. The sensing plane emitted by the transducer 10 may be parallel to a long axis of the cover 11. The cover 11 may be hollow such as to enclose the transducer 10 and extends longitudinally relative to a central axis A from an upper end 11A located adjacent to the connecting end 10A of the transducer to a lower end 11B located adjacent to the sensing end 10B of the transducer 10. The cover 11 may be contained between the connecting end 10A and the sensing end 10B of the transducer 10. The guiding system 20 may be used in conjunction with any apparatus configured for medical imaging along a sensing plane, e.g., transducers differing from that shown as transducer 10. It will be appreciated that although the cover 11 and the transducer 10 are described as being separate components, this need not be the case and in an alternative embodiment, the transducer 10 may be equipped with the cover 11 as an integral part thereof. Hence, the cover 11 and the transducer 10 may be parts of single monolithic body and hence the cover 11 may not be referred to as a cover. For simplicity, reference made herein to the transducer 10 covers embodiments in which the cover 11 is an add-on or an integral part of the transducer 10.

The guiding system 20 is configured to be removably secured to the cover 11 of the transducer 10. The guiding system 20 may alternatively be part of the transducer 10 (i.e., non-removable) as described above. The guiding system 20 includes a reference member 30 secured to the cover 11 of the transducer 10 and a guiding member 40 engageable to the reference member 30 and configured for guiding the surgical tool (e.g., the needle or catheter). In some embodiments, the reference member 30 is removably secured to the cover 11 of the transducer 10. However, in some embodiments, the reference member 30 may be defined by the cover 11. In other words, the cover 11 and the reference member 30 may be parts of a single monolithic component of the transducer 10, or a single integral apparatus. The reference member 30 is described below using reference numerals in the 30's while the guiding member 40 is described below using reference numerals in the 40's. The surgical tool may be, for instance, a needle, a catheter, scissors, scalpel, thread guide, or any other tools used during surgeries.

In some embodiments, the reference member 30 includes a collar 31 extending around the central axis A. The collar 31 may or may not extend a full circumference around the central axis A to define an opening via which the collar 31 may be engaged to the cover 11 of the transducer 10. The collar 31 is used to removably secure the reference member 30 to the cover 11 of the transducer 10. The reference member 30 includes at least one, three in the embodiment shown, attachment member 32. The three attachment members 32 are spaced apart from one another by 90 degrees about the central axis A. The attachment members 32 are circumferentially spaced apart from one another about the central axis A and are mounted to the collar 31. Each of the attachment member 32 defines a reference face 32A facing away from the central axis A. The reference faces 32A each define a reference surface for the guiding member 40 as will be explained below. Each of these reference faces 32A are co-planar with the guiding member 40 when engaged to it, and may be circularly shaped (i.e., circular) to facilitate rotation of the guiding member 40 relative to the attachment members 32 as will be discussed below. Other shapes may be used.

In the depicted embodiment, each of the attachment members 32 includes a magnet 33, which may be of substantially flat cylindrical shape, but any suitable shape is contemplated. More than one magnet may be used (e.g., a collection of small magnets). The magnet 33 is received within a cavity 32B defined by the attachment member 32. The attachment member 32 may define a slot 32C via which the magnet 33 may be inserted, but this is only optional as the magnet 33 may be embedded therein. The slot 32C may allow the replacement of the magnet 33 if need be. In some embodiments, electromagnets may be used. In some cases, the attachment members 32 may be magnet themselves. In other words, the reference face 32A may be defined by one or more magnet.

The transducer 10 may be used to perform two types of alignments of the surgical tool: an in-plane alignment and an out-of-plane alignment. For in-plane alignment, the surgical tool (e.g., needle or catheter) is aligned with a long axis of the transducer 10 or with an ultrasonic beam, along an ultrasonic beam emitted by the transducer 10 such that the tool is visible along at least a portion of its length, including its tip. For the out-of-plane alignment, the tool and the long axis of the transducer 10 (or other axis of reference) are perpendicular to one another such that a cross-section of the tool is visible. The tip of the tool may not be visible using the out-of-plane alignment.

The reference member 30 includes three attachment members 32 each for a specific use. Namely, the three attachment members include a left-side in-plane attachment member 132, a right-side in-plane attachment member 232, and an out-of-plane attachment member 332. The left- and right-side in-plane attachment members 132, 232 are opposite one another and located such that the reference faces 32A are parallel to the long axis of the transducer 10. Put differently, the reference faces 32A of the two in-plane attachment members 132, 232 face directions being transversal to a scanning plane of the ultrasonic transducer 10. The scanning plane is parallel to a long side of the ultrasonic transducer 10. Hence, the left- and right-side in-plane attachment members 132, 232 are used for in-plane alignment of the surgical tool. Two in-plane members 132, 232 are provided such both of a left-handed practitioner and a right-handed practitioner may comfortably use the guiding system 20.

The out-of-plane attachment member 332 is located between the left- and right-side in-plane attachment members 132, 232 and its reference face 32A is intersected by the long axis of the transducer 10. The out-of-plane attachment member 332 is used for out-of-plane alignment of the tool. The out-of-plane attachment member 332 faces a direction being parallel to the scanning plane of the ultrasonic transducer 10. In other words, the reference face 32A of the out-of-plane attachment member 332 is perpendicular to the scanning plane.

Still referring to Fig. 1, the guiding member 40 includes a sliding section 41 and a guiding section 42. The sliding section 41 and the guiding section 42 are secured to one another via a connecting section 43 that defines an elbow or other offsetting formation. In the embodiment shown, the sliding section 41 and the guiding section 42 are parallel to one another, but offset from one another. In other words, these two sections are not co-planar, but they may be coplanar in some embodiments. As will be described below, a holding mechanism 200 may be used to interconnect the catheter or other surgical tool to the guiding member 40.

The connecting section 43 is used to create the offset between the sliding section 41 and the guiding section 42 of the guiding member 40. A distance L1, taken as a normal to a plane of the sliding section 41, and between the sliding section 41 and the guiding section 42 of the guiding member 40 is selected such that the guiding section 42, and the surgical tool (e.g., the needle or catheter) secured thereto, is aligned and parallel to the long axis of the transducer 10 (or other reference axis) when using either one of the left- and right-side in-plane attachment members 132, 232, or such that the tool is transversally intersected by said long axis when using the out-of-plane attachment member 332. Moreover, the distance L1 may optionally be selected such that the surgical tool is aligned with a center of the scanning plane when the reference member 40 is mounted to the out-of-plane reference member 332. As will be discussed below, this distance L1 may be created by another component, namely, a holding mechanism. This may allow the guiding and connecting sections to be coplanar.

A magnetic connection is used to magnetically connect the sliding section 41 of the guiding member 40 to one of the attachment members 32 of the reference member 30. The magnetic connection may be created by a magnet mounted to one of the attachment member 32 and the sliding section 41 of the guiding member 40 and a magnetically-attractable member mounted to the other of the attachment member 32 and the sliding section 41 of the guiding member 40. The sliding section 41 includes a magnetically-attractable plate 44. In the embodiment shown, the magnetically-attractable member corresponds to the magnetically-attractable plate 44. However, the magnetically-attractable member may be another magnet with polarities aligned such that the two magnets attract one another. In some embodiments, one or two electromagnet(s) may be used. A reverse arrangement is possible, with the magnet being in the guiding member 40 and a magnetically-attractable member being in the attachment member(s) 32 (e.g., ferromagnetic material).

In this embodiment, the magnetically-attractable plate 44 is shaped as a rectangle with rounded extremities. Other shapes are contemplated, such as a capsule shape. The extremities may be semi-circular. A shape of the plate 44 may be circular. A diameter of these rounded extremities substantially matches or is greater than a diameter of the reference faces 32A of the attachment members 32 of the reference member 30. The sliding section 41 defines a sliding reference face 44A, which is herein defined by the magnetically-attractable plate 44.

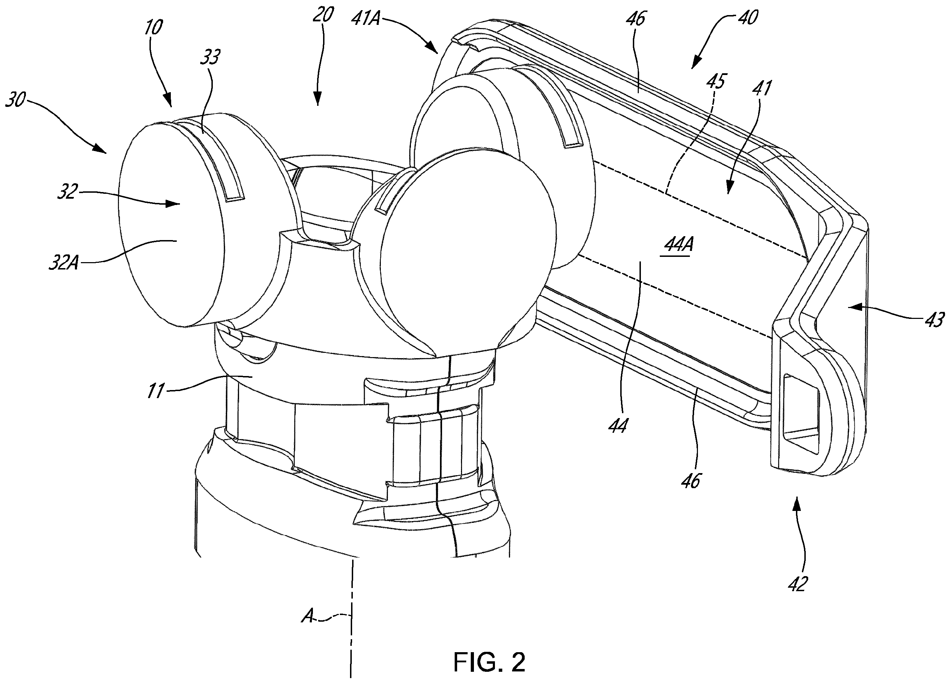

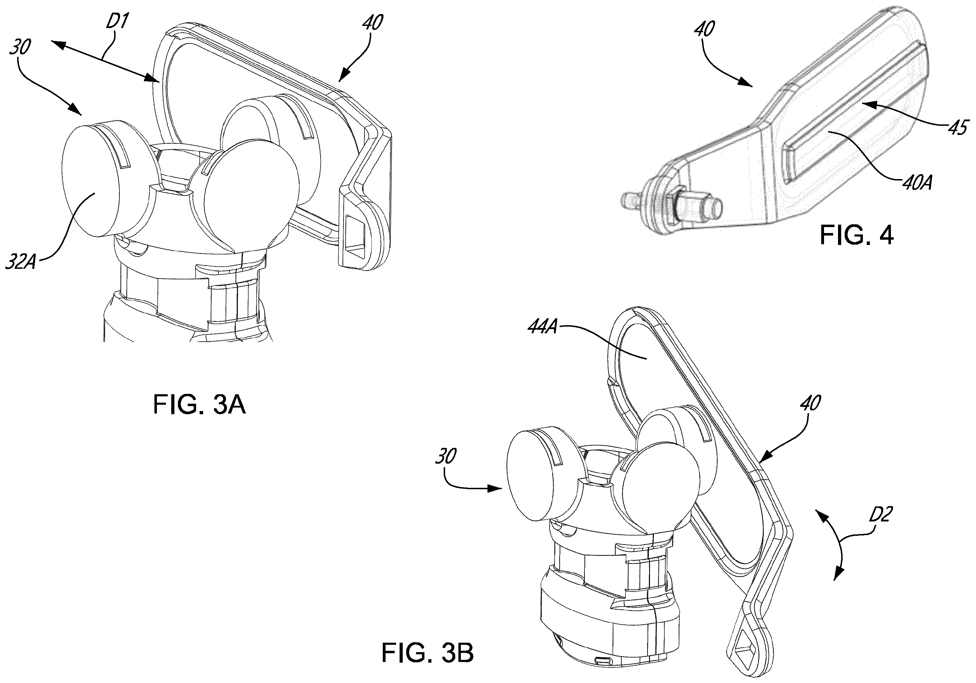

Referring more particularly to Fig. 2, the guiding member 40 is shown engaged to one of the attachment members 32. In this embodiment, the guiding member 40 is magnetically connected to the one of the attachment members 32. In the context of the present disclosure, the expression "magnetically connected" implies that the guiding member 40 and the one of the attachment members 32 are secured to one another via magnetic attraction created by the magnets 33 of the attachment members 32. Hence, in some embodiments, the magnetically-attractable plate 44 is made, for instance, of a ferromagnetic material. It will be appreciated that any suitable combinations of magnet and magnetically-attractable material may be used. For instance, the attachment members 32 may be devoid of a magnet and the reference face 32A may be defined by a magnetically-attractable plate and the magnet may be provided on the guiding member 40. This plate may be alternatively embedded into the attachment members 32. In some embodiments, both of the sliding section 41 and the attachment members 32 may include a respective magnet with polarities aligned to ensure that the guiding member 40 and the attachment members 32 attract one another. In the embodiment shown, the guiding member 40 further includes a longitudinal or elongated magnet 45, which is shown in dashed line in Fig. 2. As shown more clearly in Fig. 4, the longitudinal magnet 45 may be rectangularly shaped or oblong and extends substantially a length of the sliding section 41 of the guiding member 40. In this configuration, the longitudinal magnet 45 is received into a sleeve 40A defined by the guiding member 40. This sleeve 40A may permit periodic replacement of the longitudinal magnet 45. In the context of the present disclosure, the expressions "substantially" and "about" implies variations of plus or minus 10%. The longitudinal magnet 45 may sit behind the plate 44, which, in this case, does not need to be made of a magnetically-attractable material, but may be made of plastic or any material to facilitate sliding of the sliding section 41 against the reference face 32A of the attachment members 32 of the reference member 30.

The guiding member 40 further includes rails 46, two rails 46 in the embodiment shown, extending lengthwise along the sliding section 41 and being substantially parallel to the longitudinal magnet 45. The plate 44 is disposed between the two rails 46. The rails 46 protrude transversally from the reference face 44A and away therefrom. The rails 46 are used to maintain the reference face 32A of the attachment member 32 in contact with the sliding reference face 44A of the guiding member 40 during movements of the guiding member 40 relative to the reference member 30. Put differently, the rails 46 define abutments for contacting the attachment members 32 to prevent them from separating from the plate 44. The rails 46 extend parallel to one another and end at a distal end of the sliding section 41 to define an opening 41A via which the attachment member 32 may be freed from its magnetic attraction to the sliding section 41. The opening 41A is thus free or devoid of the rails 46. The rails 46 end at the opening 41A. The rails 46 may be made of a material that is not magnetically attracted by the reference members 32, such as plastic.

Referring to Figs. 3A and 3B, motions of the guiding member 40 relative to the reference member 30 are illustrated. As shown in Fig. 3A, the guiding member 40 may be translated in a lengthwise direction D1 along the length of the sliding section 41. As shown in Fig. 3B, the guiding member 40 may be rotated along direction D2 relative to the reference member 30. It will be appreciated that any compound movements are possible, such as a combination of a translation along the direction D1 and a rotation along the direction D2 while the guiding member 40 and one of the attachment members 32 are magnetically connected to one another. Put differently, these movements in a translational degree of freedom (DOF) and a rotational DOF are possible while the sliding reference face 44A of the plate 44 is in contact with the reference face 32A of the one of the attachment members 32. In some embodiments, up and down motion of the guiding member 40 relative to the reference member 30 is possible by about a few millimetres (e.g., from 0 to 1 cm) since the diameter of the reference face 32A is less than a height of the sliding reference face 44A. Hence, movement is optionally possible in 2 translational DOFs along with the rotational DOF.

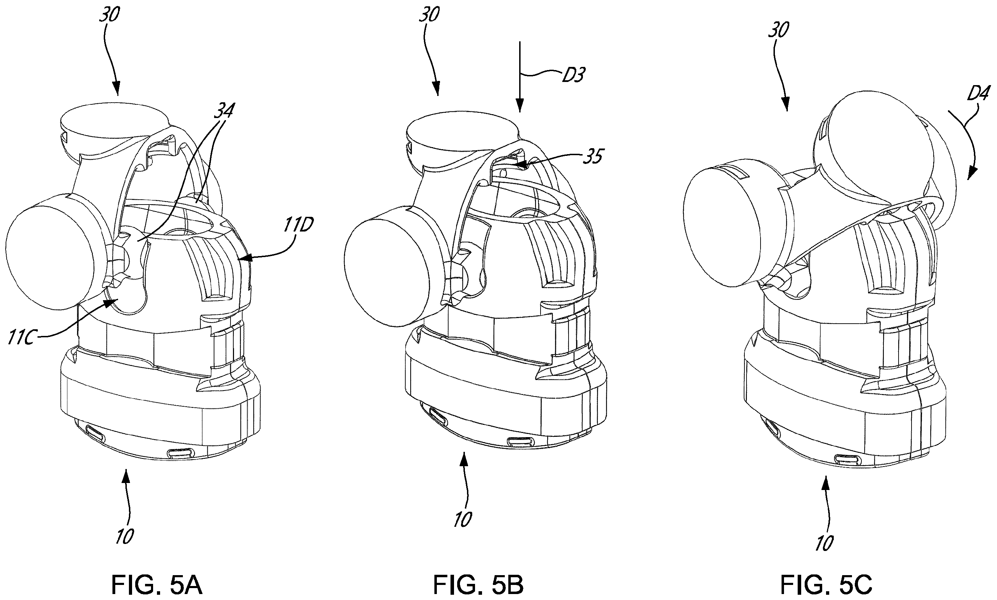

Referring to Figs. 5A-5C, steps of assembling the reference member 30 to the transducer 10 are illustrated. It will be appreciated that other assembly steps may be required in some embodiments. Other embodiments may necessitate other assembling steps.

In the embodiment shown, the cover 11 of the transducer 10 defines two lateral grooves 11C each located on opposite sides thereof and defines a rib 11D located circumferentially between the two lateral grooves 11C. The reference member 30 is configured to mate with these features of the transducer 10. Understandably, the reference member 30 may be made with any suitable feature used to mate with the transducer 10. In this embodiment, the reference member 30 includes two protrusions 34 each secured to the collar 31 at opposite ends therefor and defines a slot 35 defined by the collar 31 between the two protrusions 34.

As shown in Fig. 5A, to assemble the reference member 30 to the transducer 10, the collar 31 is first oriented to be substantially vertical and each of the two protrusions 34 is inserted into a respective one of the two lateral grooves 11C. As shown in Fig. 5B, the collar 31 is pushed along direction D3, which is substantially parallel to the central axis A of the transducer 10, until the protrusions 34 contact abutments located at ends of the two lateral grooves 11C. Then, as shown in Fig. 5C, the collar 31 is rotated along direction D4 about an axis intersecting both of the two protrusions 34. This rotation is permitted by the protrusions 34 and the abutments at the end of the grooves 11C being rounded. The collar 31 is rotated along the direction D4 until the rib 11D is received into the slot 35. These movements are optional, as the reference member 30 may be fixed or integral to the transducer 10.

The disclosed guiding system 20 may facilitate the insertion of a needle or catheter in a blood vessel of a patient. The needle or catheter may remain sterile before any contact with the patient. The system 20 permits two types of procedures: in-plane and out-of-plane alignments to cater to the preferences of the medical practitioner.

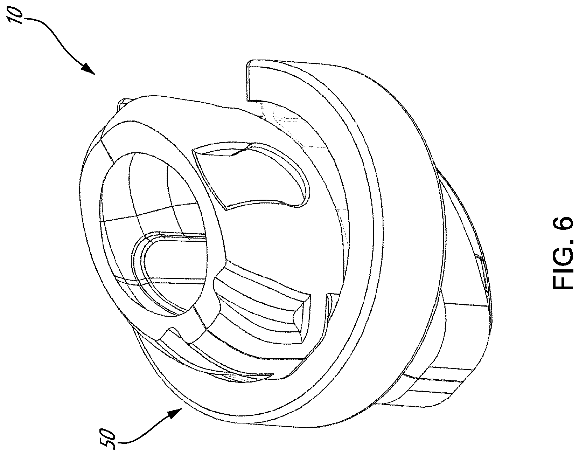

Referring now to Fig. 6, the transducer 10 is shown equipped with a rotating mechanism 50, which is optional. The rotating mechanism 50 may be releasably secured to the cover 11 of the transducer 10 and configured to permit rotation of the transducer 10 relative to the rotating mechanism 50. In some embodiments, the rotating mechanism 50 may be integrated to the cover 11 of the transducer 10. This may facilitate some operations performed by the medical practitioner using the transducer 10 as will be described below.

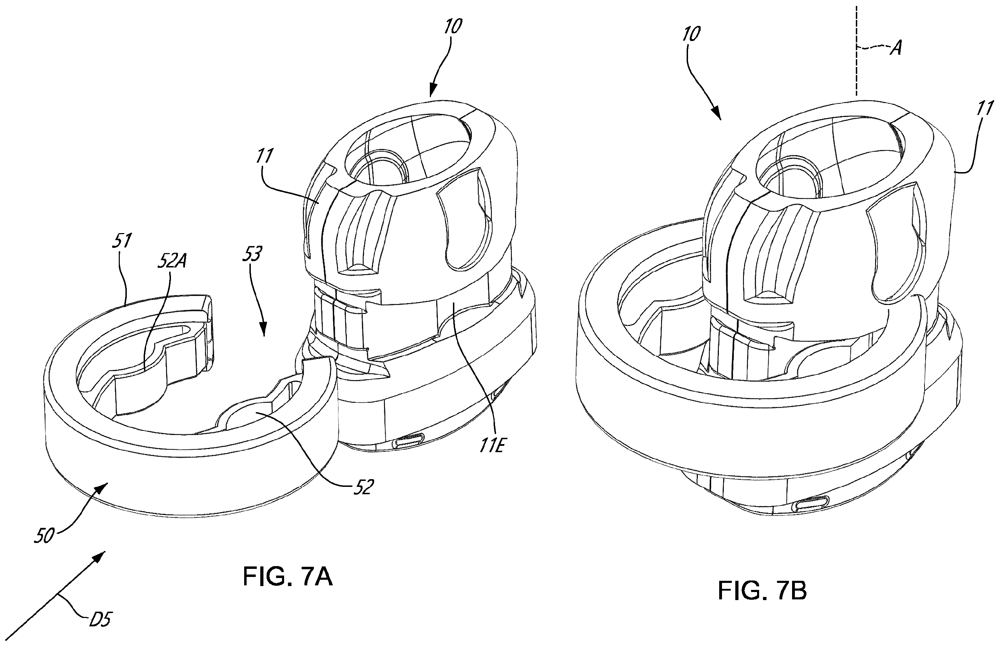

Referring to Fig. 7A, the rotating mechanism 50 includes an outer member 51 and an inner member 52. The outer member 51 is rotatable relative to the inner member 52 about the central axis A of the cover 11 of the transducer 10 as will be described below. In the embodiment shown, the inner member 52 and the outer member 51 define an opening 53 via which the rotating mechanism 50 may be coupled to the cover 11 of the transducer 10. Also, this opening 53 may allow a practitioner to wrap a sterile bag around the rotating mechanism 50. The opening 53 may allow the rotating mechanism 50 to clip to the transducer 10 even if the latter is wrapped in a sterile bag. Understandably, the openings of both members need to be aligned circumferentially to be able to mount the rotating mechanism 50 to the cover 11 of the transducer 10. The inner member 52 defines two bumps 52A that are sized to mate with correspondingly-shaped recesses 11E defined on an outer surface of the cover 11 of the transducer 10. The two recesses 11E are located on opposite sides of the cover 11 and on a long side of said cover 11. The recesses 11E may alternatively be on a short side of the cover 11 in other embodiments.

Referring to Figs. 7A and 7B, an assembly procedure of the rotating mechanism 50 on the cover 11 of the transducer 10 is shown. It is understood that this assembly procedure is exemplary and may vary with other embodiments of the rotating mechanism 50. In Fig. 7A, the cover 11 is inserted inside the rotating mechanism 50 via the opening 53 along direction D5, which is transverse to the central axis A of the cover 11. As shown in Fig. 7B, force is exerted along the direction D5 until the bumps 52A are in register with the recesses 11E of the cover 11. The outer and inner members 51, 52 have resiliency to be elastically deformable to permit the insertion of the cover 11 into an inner cavity of the rotating mechanism 50 until the bumps 52A snap into engagement with the recesses 11E. It will be appreciated that other means of securing the inner member 52 to the cover 11 may be used such as, for instance, fasteners, keyway, dog and slot, and so on.

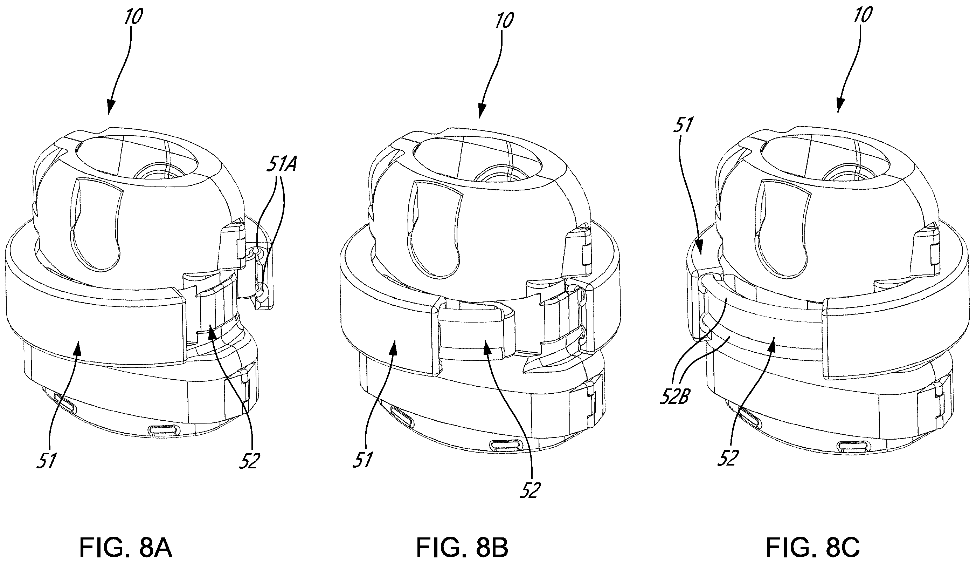

Referring now to Figs. 8A to 8C, different positions of the inner member 52 relative to the outer member 51 are illustrated. As illustrated, the inner member 52 is received within a groove 51A defined by the outer member 51. More specifically, in the embodiment shown, the inner member 52 includes two arcuate rails 52B each slidably received within a respective one of two arcuate grooves 51A of the outer member 51. The arcuate rails 52B and the arcuate grooves 51A extend circumferentially around the central axis A and define an arcuate shape, such as a circular shape permitting the relative rotation between the inner member 52 and the outer member 51.

The rotating mechanism 50 may be used in conjunction with the guiding system 20 described above with reference to Figs. 1 to 5C. During use, the practitioner may hold the cover 11 of the transducer 10 with the rotating mechanism 50, more specifically with the outer member 51, until the medical practitioner finds the parts of the body he/she is looking for (e.g., blood vessel). In searching for this blood vessel, the transducer 10 may be positioned such that the scanning plane intersects the blood vessel either along its length or across its length. When the blood vessel is located, the practitioner may wish to rotate the scanning plane 90 degrees before proceeding with the insertion of the surgical tool, in this case a needle or catheter. However, movements of the hand may be imprecise. Therefore, the practitioner continues to hold the transducer 10 via the outer member 51 with the same hand while the other hand rotates the transducer 10. This movement is permitted by a rotation of the cover 11 of the transducer 10 and of the inner member 52 relative to the outer member 51. The hand that holds the outer member 51 maintains the proper alignment of the transducer 10 while the other hand rotates it. In some cases, the hand that causes the rotation may be positioned on the guiding member 40 or on the reference member 30. When the rotation is completed, the user may squeeze the outer member 51 thereby frictionally blocking rotation of the outer member 51 relative to the inner member 52. This allows the practitioner to maintain the alignment with the blood vessel.

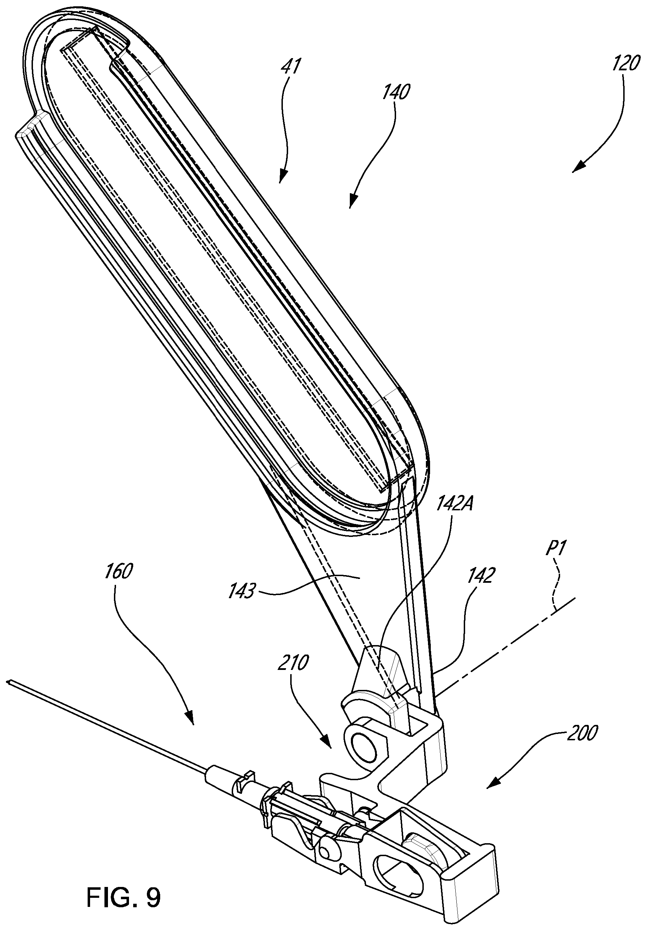

Referring to Fig. 9, another embodiment of a guiding system is shown at 120. For the sake of conciseness, only features differing from the guiding system 20 described above are described below. The guiding system 120 includes a guiding member 140, similar to the guiding member 40 described above, with some differences that are explained below. The guiding member 140 is configured to engage a needle or catheter, denoted at 160. A holding mechanism 200 is configured to interconnect the catheter 160 to the guiding member 140. These different features are described, one after the other, below. The holding mechanism 200 is also configured to allow a medical practitioner to manipulate the catheter 160 while maintaining proper alignment as will be described below. In a variant, an Aspectic No Touch Technique (ANTT) is used. The holding mechanism 200 disclosed herein may allow to meet requirements of an ANTT procedure, namely a technique in which a user may not touch the patient's skin. Holding mechanism 200 includes a connecting member 210 to be pivotably engaged to the guiding member 140, and a holder 220 detachably securable to the connecting member 210 and configured for holding the catheter 160. The holding mechanism 200 may further allow the disconnection of the guiding system 20 from the catheter 160 while meeting requirements of the ANTT procedure. Indeed, the practitioner may unclip or otherwise detach the catheter 160 from the guiding system 20 while the catheter 160 is inserted in the patient. This may be done by unclipping the holder 220 and catheter 160 mounted thereto from the connecting member 210 while being free of contact with the patient.

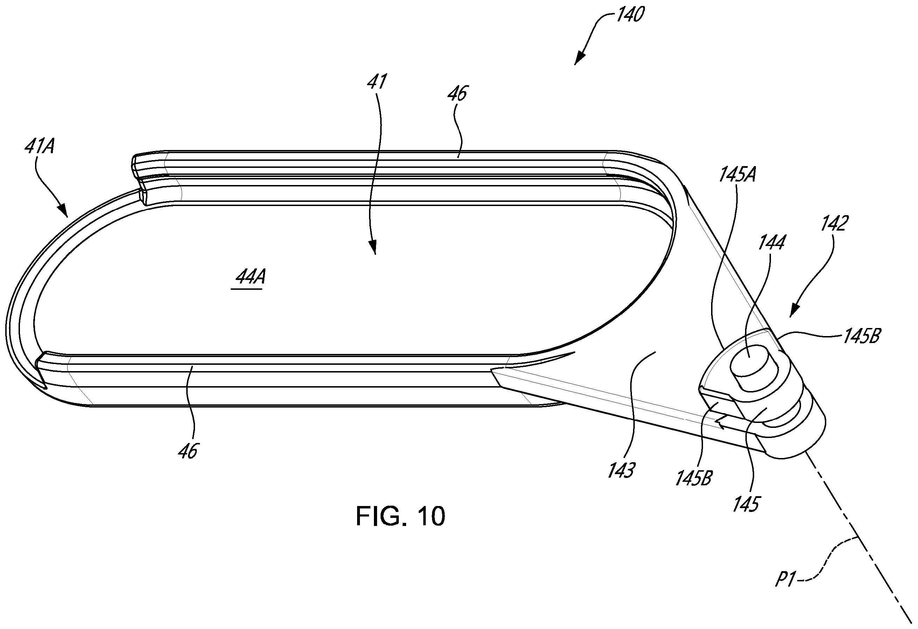

Referring to Fig. 10, the guiding member 140 is shown in greater detail. In this embodiment, the guiding member 140 includes a sliding section 41 as described above. The sliding section 41 is connected to a connecting section 143 that leads to a guiding section 142. In this embodiment, the connecting section 143 and the sliding section 41 are substantially co-planar and/or parallel, although a small offset may be present. This contrasts with the configuration of Fig. 1 that shows that the sliding section 41 and the guiding section 42 are not co-planar and offset from one another.

As will be described below, the guiding section 142 pivotably engages the holding mechanism 200 for relative rotation about a pivot axis P1. The guiding section 142 includes a shaft 144 extending transversally to the connecting section 143. A locking member 145 is removably engageable to the shaft 144. In some embodiments, the locking member 145 may be frictionally engaged to the shaft 144 via a bore defined therethrough. Alternatively, the locking member 145 may be threadingly engaged to the shaft 144 via correspondingly-mating threads. Other configurations are contemplated (e.g., an indexing of the locking member 145). The shaft 144 is used to define a pivot for rotation of the guiding member 140 relative to the holding mechanism 200. Any suitable means may be used for that purpose. Bearings may be used in some embodiments. The locking member 145 may have an arcuate surface 145A configured to engage an arcuate tab 142A (Fig. 9) of the guiding member 140. The arcuate surface 145A and the arcuate tab 142A may slide against one another such as to constraint the movement to a rotation about the pivot axis P1. Moreover, the locking member 145 may define two abutment faces 145B configured to abut a part of the holding mechanism 200 to limit a range of motion of the guiding member 140. For instance, as shown in Fig. 9, the two abutment faces 145B may abut a connecting member 210 described below of the holding mechanism 200 to limit the range of motion. However, it will be appreciated that this may not be required and that a full free rotation of the guiding member 140 about the holding mechanism 200 may be possible. The locking member 145 is used to limit the rotation of the connection member 210 described below within a desired range.

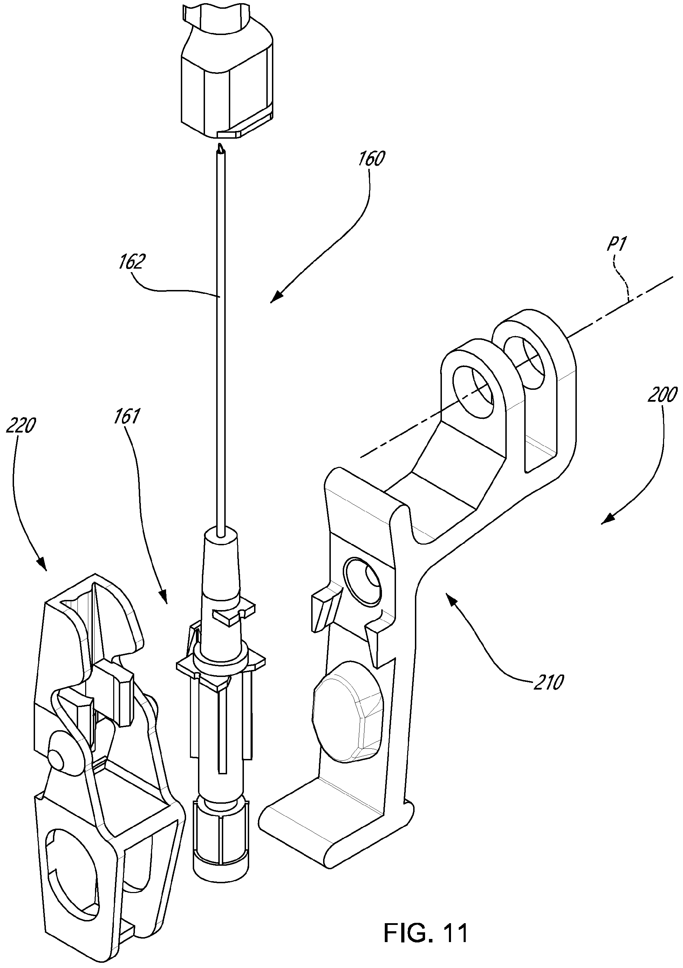

As shown in Fig. 11, the catheter 160 includes a base 161, via which a source of a fluid to be injected, may be secured. The base 161 is configured to be connected via any other suitable devices. The catheter 160 has a tip 162 configured to pierce through patient soft tissue (e.g., going through tissue).

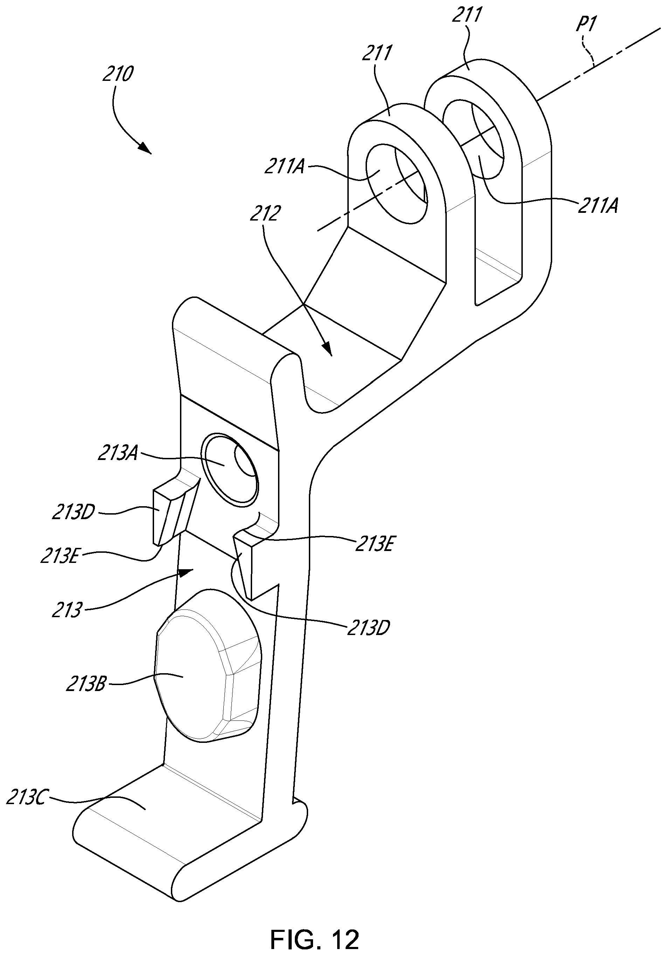

Referring to Figs. 11-12, the holding mechanism 200 is pivotably engaged to the connecting section 143 of the guiding member 140. To that end, the holding mechanism 200 includes a connecting member 210 pivotably engaged to the guiding section 142. The connecting member 210 includes a body defining two brackets 211, although only one or more than two may be used. The two brackets 211 define each an aperture 211A in register with one another and sized to receive therein the shaft 144 of the guiding member 140. A space defined between the two brackets 211 is sized to receive therein the locking member 145. It will be appreciated that any means used for pivotably connecting the connecting member 210 to the guiding member 140 may be used. For instance, a tube and a shaft received in the tube may be used, a hinge, and so on. The connecting member 210 further includes a spacer 212 and a support member 213. The two brackets 211 extend transversally to the spacer 212 and the support member 213 extends transversally to the spacer 212, but other arrangements are possible. The brackets 211 and the support member 213 may extend away from one another relative to the spacer 212, i.e. in opposite directions relative to the spacer 212. The spacer 212 is configured to create an axial offset between the sliding section 41 of the guiding member 140 and the catheter 160. As explained above, a length of the spacer 212 is selected to ensure that the catheter 160 is intersected by a scanning plane of the transducer 10. The support member 213 is configured to hold the needle as will be explained further below. The spacer 212 may be engaged by a hand of the medical practitioner as will be detailed below. The different parts of the spacer 212 have been described as belonging to a single monolithic body, but this need not be the case and the spacer 212 may be created by assembling a plurality of distinct parts.

The support member 213 defines a dimple 213A, which has a frustoconical shape, but other shapes are contemplated, and other configurations, including a hole, a bore, etc. The dimple 213A may be used to facilitate an engagement with another element of the holding mechanism 200 that will be described below, in male-female engagement as a possibility among others. The dimple 213A may be seen as a recess or cavity defined by the support member 213. The support member 213 further defines a protrusion 213B protruding substantially axially relative to the pivot axis P1 and away from the two brackets 211, but this is optional. The protrusions 213B may have a tapered shape such that its cross-sectional area taken on a plane normal to the pivot axis P1 decreases from its base towards its tip. The protrusion 213B may have a non-circular shape (e.g., oval, ellipse, etc), which may be used to impede rotation of a mating element, but this may not be necessary in all embodiments. The connecting member 210 may include a rear tab 213C (a.k.a., trailing tab, relative to an insertion direction of the needle) secured, and extending transversally, to an end of the support member 213. The rear tab 213C is located such as to be oriented towards a base of the needle and may be engaged by a hand of the medical practitioner to manipulate the catheter 160 mounted thereto. In other words, a longitudinal axis of the needle of the catheter may be normal to the rear tab 213C. The support member 213 further defines two abutments 213D protruding substantially axially relative to the pivot axis P1 and away from the two brackets 211. It will be appreciated that other means of removably engaging the connecting member 210 to the holder 220 are contemplated. Hence, the abutments 213D may be omitted and replaced by other features. The two abutments 213D are angled such as to both face each other and partially face towards the protrusion 213B. In other words, the two abutments 213D are positioned to form a V-shape. The two abutments 213D converge towards one another in a direction parallel to a longitudinal axis of the support member 213. The two abutments 213D may be parallel to one another in some configurations. The two abutments 213D each define a bulge 213E at or proximate a tip thereof.

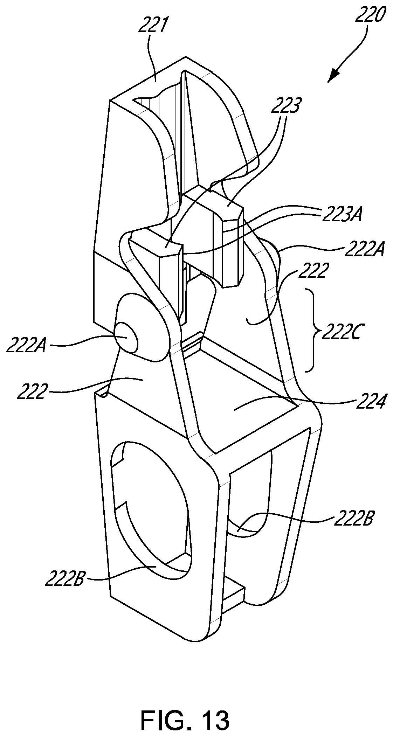

Referring to Fig. 13, the holding mechanism 200 further includes a holder 220 that is detachably securable to the connecting member 210, though it could be an integral part thereof. The holder 220 has a base 221 and two lateral walls 222 being spaced apart from one another and extending transversally from the base 221. The two lateral walls 222 may or may not be non-parallel to one another and may extend towards one another in a direction oriented towards the tip 162 of the catheter. This may contribute in increasing a stability of the catheter 160, but may not be present in all configurations. The two lateral walls 222 define a space therebetween configured for receiving the catheter 160. Each of the two lateral walls 222 defines a respective bump 222A extending away from one another and configured to engage the dimple 213A. The dimple 213A and the bump 222A may be said to have mating geometries, one being the "negative" of the other. The engagement of the bump 222A into the dimple 213A may create a positive stop between these two elements. Also, the bump 222A that is not received in the dimple 213A may be engaged by a finger of the medical practitioner. This may assist the practitioner in manipulating the catheter 160. The bumps may be omitted in some embodiments. The two lateral walls 222 also each defines an aperture 222B sized to receive therethrough the protrusion 213B of the support member 213. Because of the tapered shape of the protrusion 213B described above, the engagement of the protrusion 213B in the aperture 222B may create a tight fit limiting play between these components. It will be appreciated that this protrusion may not be necessary in all configurations.

The two lateral walls 222 each optionally have a flared portion 222C of decreasing height and converging towards the bumps 222A. The height is taken along a direction being transverse to the base 221. The flared portions 222C are shaped to be received between the two abutments 213D of the support member 213. The holder 220 may include a back wall 224 that may be in abutment against the catheter 160 when engaged to the holder 220. This may further provide a secure fit of the catheter 160 to the holder 220. The back wall 224 may further increase a stiffness of the holder 220 by limiting deformation of the lateral walls 222 when engaged by a medical practitioner. Moreover, the flared portion 222C, by decreasing in height, may contribute in securely being held by the connecting member 210. The converging shape of the flared portion 222C of the lateral walls 222 may cause an inward stabilizing force as the practitioner manipulates the catheter 160. It can be observed that the holder 220 is symmetrical, such that it may be connected from either side to the connecting member 210. A similar observation may be made regarding the connecting member 210. Therefore, the same pair of connecting member 210 and holder 220 may be used with the transducer 10 and guiding system 20/120, regardless of the side. However, it is possible to have side-specific sets of connecting member 210 and holder 220.

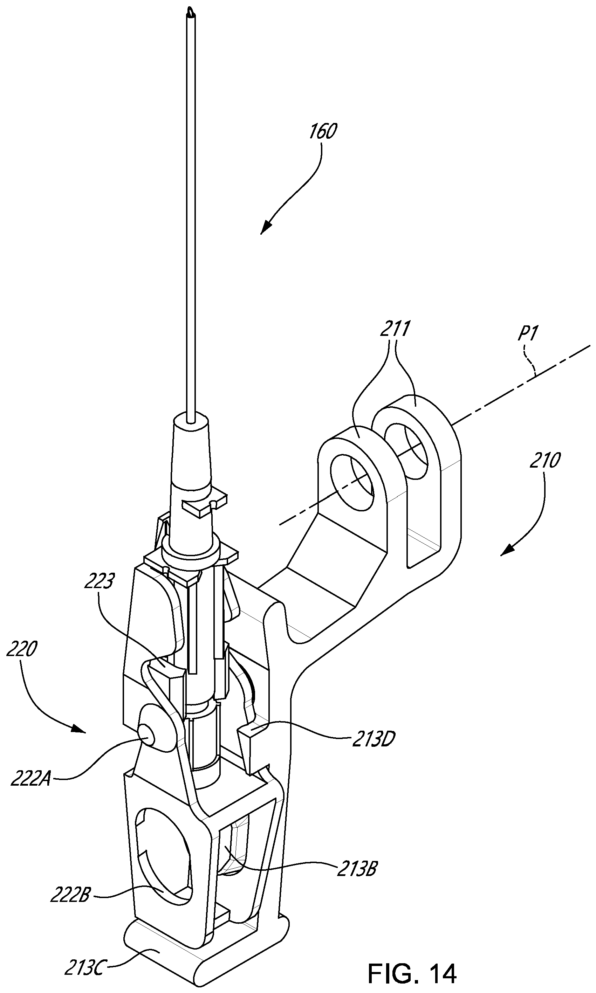

Referring to Figs. 13-14, when it is desired to mount the holder 220 to the connecting member 210, these two components are moved towards one another until the protrusion 213B is received into one of the apertures 222B of the two lateral walls 222. At which point, a force may be applied to move these to components towards one another to slightly resiliently deform the two 213D abutments with contact against the bulges 213E until the holder 220 snap fits in engagement with the connecting member 210. At which point, one of the bumps 222A is received into the dimple 213A, or other mating engagement is achieved. This snap fit connection or equivalent may provide an efficient way and reproducible way of releasably engaging and disengaging these two components together. Moreover, the engagement of the protrusion 213B into one of the apertures 222B and of one of the bumps 222A into the dimple 213A may stabilize these two components together, which may effectively remove any "play" that may otherwise exist between them. This may ensure a precise and accurate manipulation of the catheter 160. It will be appreciated that other securing means may be used, such as fasteners, dog and slot, bayonet lock, keyway engagement, and so on. In this embodiment, the holder 220 may be engaged and disengaged from the connecting member 210 in a direction being solely axial relative to the pivot axis P1. Indeed, the holder 220 may be blocked from moving in other directions via the rear tab 213C and the two abutments 213D. This may promote a safe disconnection of the holder 220 from the connecting member 210 while minimizing risks incurred by manipulating a used catheter. It is understood that, in other embodiments, the holder 220 may be separated from the connecting member 210 along other directions (e.g., parallel to the catheter 160, rotation of the holder 220 relative to the connecting member 210.

Referring back to Fig. 13, in the embodiment shown, the holder 220 further includes two needle-engaging tabs 223 protruding transversally from the base 221 and located between the two lateral walls 222. The tabs 223 are spaced apart form one another suitably to receive the base 161 of the catheter 160 therein as described below. The tabs 223 define bulges 223A at their tips to provide a snap fit connection with the base 161 of the catheter 160. The tabs 223 may resiliently deform by flexion away from one another when pushing the catheter 160 between them. Put differently, the tabs 223 are used to secure the catheter 160 to the holder 220. It will be understood that that any other ways of securing the catheter to the holder 220 may be used. This may include, a strap, a bracket, a fastener, and so on. In some embodiments, the holder 220 may be undetachable from the catheter 160 without creating damage to one or both of the catheter 160 and holder 220, in a tamper-evident manner. This may thus require discarding both of the holder 220 and catheter 160. The holder 220 may be a single use device, to be replaced after each use, while the connecting member 210 may or may not be single-use. This may be done by ensuring one or both of the tabs 223 break after removal of the catheter 160, for instance.

In some embodiments, the disconnection of the holder 220 from the connecting member 210 may be performed while complying with the Aseptic Non Touch Technique (ANTT). Specifically, the guiding system may be configured such that the practitioner can perform the disconnection without touching the area near the insertion site or the body of the needle, thus minimizing contamination risks. The geometry of the holder 220 and the support member 213 may facilitate this disconnection through one or more designated gripping areas that are located away from the patient's skin or insertion zone. Additionally, various configurations are contemplated wherein one or more auxiliary components, such as a disconnection tool, cap, lever, or any disconnection mechanism may be temporarily or permanently attached to the system to assist in the ANTT-compliant disconnection of the holder 220 from the connecting member 210. Throughout the disconnection process, the guiding system may ensure that the catheter 160 remains stable relative to the insertion site. This stability may prevent inadvertent movement or displacement of the catheter during disengagement. The disconnection may be performed using one of the following methods: (A) Frontal approach, where the practitioner faces the insertion site and performs a direct forward motion; (B) Vertical oblique approach, where the practitioner performs a disconnection in a vertical trajectory within a sagittal or oblique plane. These methods may enable compliance with clinical protocols while maintaining user ergonomics and minimizing the risk of catheter displacement. It should be understood that while particular disconnection approaches have been illustrated and described herein, other configurations and methods of detaching the holder from the connecting member may also be employed, provided they comply with the principles of the Aseptic Non Touch Technique (ANTT) and maintain the catheter's stability during the procedure. These methods may include, but are not limited to, the use of additional components, tools, or mechanisms temporarily or permanently integrated into the system to facilitate safe, ergonomic, and aseptic disconnection. Accordingly, any detachment approach that allows the user to separate the holder from the connecting member without compromising the sterility of the insertion site and without displacing the catheter shall be considered within the scope of the present disclosure.

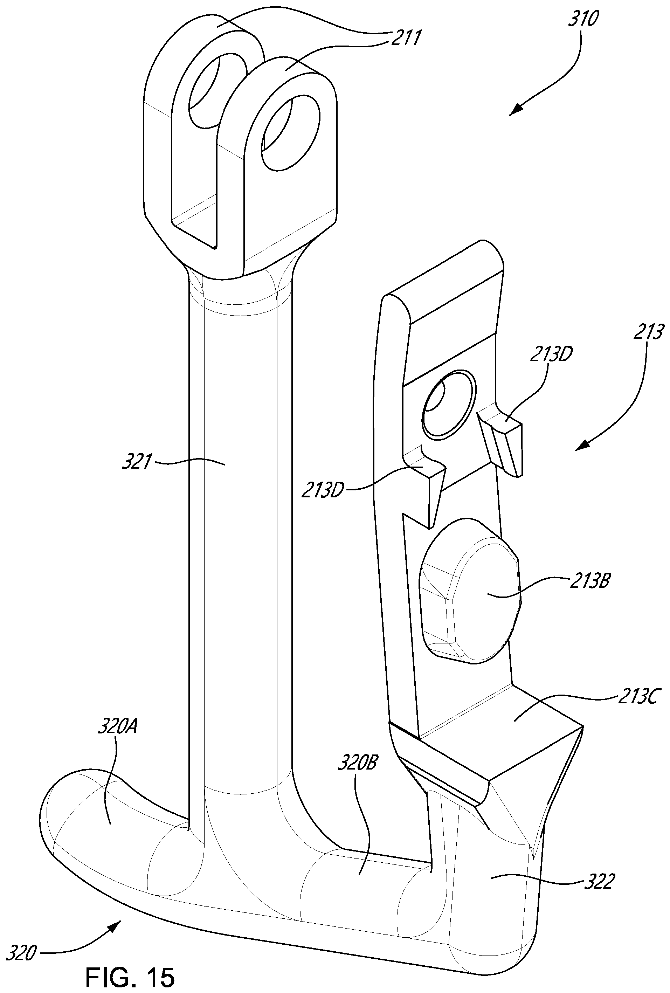

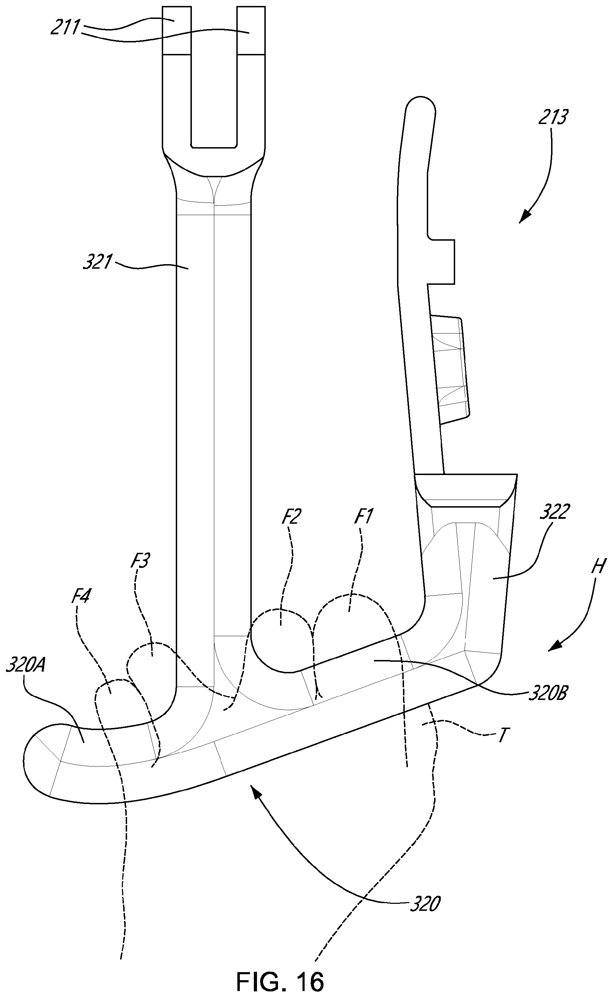

Referring now to Figs. 15-16, another embodiment of a connecting member is shown at 310. For the sake of conciseness, only features differing from the connecting member 210 described above are described below.

In the embodiment shown, the connecting member 310 also carries the support member 213 for engaging the holder 220. The connecting member 310 includes a handle 320 sized to fit within a palm of a hand H of the practitioner, though finger loops could used, such as for one or more of the fingers as detailed below. The connecting member 310 includes a longitudinal arm 321, at the end of which are located the brackets 211, or other suitable securing means, protruding transversally from the handle 320 and configured to pivotably engage the guiding member 140. The longitudinal arm 321 is offset from extremities of the handle 320 such that the arm 321 is received between two fingers of the hand H of the practitioner. Namely, in this embodiment, the arm 321 is received between fingers F2 (middle finger), F3 (ring finger) while fingers F1 (index finger), F2 are located on an opposite side of the arm 321 and are located between the arm 321 and the support member 213. Preferably, the arm 321 may be received between the fingers F3, F4 (pinky finger). In such a case, only the auricular finger is located on a left side of the arm 321. Hence, the handle 320 may have two sections 320A, 320B disposed on respective opposite sides of the arm 321, though a single one could be present. A spacer 322 may protrude form an end of the handle 320 and used to connect the support member 213 to the handle 320. It is understood this image is provided for a left-handed practitioner, but the device may be flipped 180 degrees to be used by a right-handed practitioner. This configuration may allow the practitioner to hold the connecting member 210 using the handle 320 while maintaining fine motricity ability using his or her thumb T and the finger F1 and optionally the finger F2. The fingers F3, F4 and/or the palm of the practitioner may be used to guide/rotate the connecting member 310 relative to the guiding member 140 while maintaining the guiding member 140 aligned and magnetically engaged to the reference member 30 (Fig. 2).

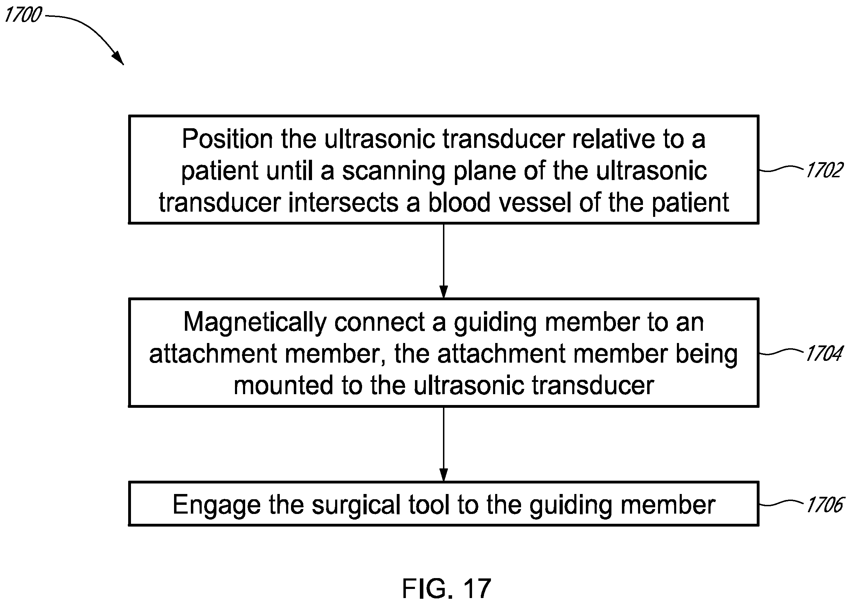

Referring to Fig. 17, a method of guiding the needle or catheter using the ultrasonic transducer 10 is shown at 1700. The method 1700 includes positioning the ultrasonic transducer 10 relative to a patient until a scanning plane of the ultrasonic transducer 10 intersects a blood vessel of the patient at 1702; magnetically connecting the guiding member 40, 140 to the attachment member 32 at 1704, the attachment member 32 being mounted to the ultrasonic transducer 10; and engaging the surgical tool to the guiding member 40, 140 at 1706.

In some embodiments, the method 1700 includes inserting the needle or catheter into the blood vessel by one or more of translating and rotating the guiding member 40, 140 relative to the attachment member 32 while the reference face 32A of the attachment member 32 remains in contact with the sliding reference face 44A of the guiding member 40 such that the needle or catheter remains either parallel to or transverse to the scanning plane of the ultrasonic transducer 10.

It is noted that various connections are set forth between elements in the preceding description and in the drawings. It is noted that these connections are general and, unless specified otherwise, may be direct or indirect and that this specification is not intended to be limiting in this respect. A coupling between two or more entities may refer to a direct connection or an indirect connection. An indirect connection may incorporate one or more intervening entities. The term "connected" or "coupled to" may therefore include both direct coupling (in which two elements that are coupled to each other contact each other) and indirect coupling (in which at least one additional element is located between the two elements).

It is further noted that various method or process steps for embodiments of the present disclosure are described in the preceding description and drawings. The description may present the method and/or process steps as a particular sequence. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the description should not be construed as a limitation.

Furthermore, no element, component, or method step in the present disclosure is intended to be dedicated to the public regardless of whether the element, component, or method step is explicitly recited in the claims. As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various aspects of the present disclosure have been disclosed, it will be apparent to those of ordinary skill in the art that many more embodiments and implementations are possible within the scope of the present disclosure. For example, the present disclosure as described herein includes several aspects and embodiments that include particular features. Although these particular features may be described individually, it is within the scope of the present disclosure that some or all of these features may be combined with any one of the aspects and remain within the scope of the present disclosure. References to "various embodiments," "one embodiment," "an embodiment," "an example embodiment," etc., indicate that the embodiment described may include a particular feature, structure, or characteristic, but every embodiment may not necessarily include the particular feature, structure, or characteristic. Moreover, such phrases are not necessarily referring to the same embodiment. The use of the indefinite article "a" as used herein with reference to a particular element is intended to encompass "one or more" such elements, and similarly the use of the definite article "the" in reference to a particular element is not intended to exclude the possibility that multiple of such elements may be present.

In the context of the present disclosure, the expression "substantially" implies small variations caused by, for instance, manufacturing tolerances. The expression "about" implies variations of plus or minus 10%.

The embodiments described in this document provide non-limiting examples of possible implementations of the present technology. Upon review of the present disclosure, a person of ordinary skill in the art will recognize that changes may be made to the embodiments described herein without departing from the scope of the present technology. Yet further modifications could be implemented by a person of ordinary skill in the art in view of the present disclosure, which modifications would be within the scope of the present technology.

## Claims

1. A guiding system for an ultrasonic transducer having a body, comprising:
a reference member releasably secured to the body of the ultrasonic transducer, the reference member having an attachment member defining a reference face;
a guiding member having:
a sliding section defining a sliding reference face, and
a guiding section connected to the sliding section, the guiding section configured to hold a surgical tool for insertion into a patient; and
a magnetic connection to magnetically connect the sliding section of the guiding member to the attachment member and to maintain the reference face of the attachment member in contact with the sliding reference face of the sliding section while allowing translation and rotation of the guiding member relative to the attachment member,
the magnetic connection created by a magnet mounted to one of the attachment member and the sliding section of the guiding member and a magnetically-attractable member mounted to the other of the attachment member and the sliding section of the guiding member.

2. The guiding system of claim 1, wherein the reference face is circular to facilitate rotation of the guiding member relative to the reference member.

3. The guiding system of claim 1 or 2, wherein the reference member includes a collar mounted at least partially around the body of the ultrasonic transducer, the attachment member mounted to the collar.

4. The guiding system of any one of claims 1 to 3, wherein the attachment member includes three attachment members disposed around the body of the ultrasonic transducer, the three attachment members spaced apart from one another by 90 degrees.

5. The guiding system of claim 4, wherein the three attachment members include two in-plane attachment members being located on opposite sides of the body of the ultrasonic transducer, the two in-plane attachment members located on a long side of the body.

6. The guiding system of claim 5, wherein one or more of:
reference faces of the two in-plane attachment members face directions being transversal to a scanning plane of the ultrasonic transducer; and
the three attachment members include an out-of-plane attachment member disposed circumferentially between the two in-plane attachment members, the out-of-plane attachment member facing a direction being parallel to a scanning plane of the ultrasonic transducer.

7. The guiding system of any one of claims 1 to 6, wherein the attachment member includes a magnet and wherein the sliding section includes a magnetically-attractable plate defining the sliding reference face.

8. The guiding system of claim 7, wherein the magnetically-attractable plate includes a second magnet configured to attract the magnet of the attachment member.

9. The guiding system of any one of claims 1 to 8, wherein the sliding section defines rails, the sliding reference face located between the rails, the rails configured to maintain the reference face of the attachment member in contact with the sliding reference face.

10. The guiding system of any one of claims 1 to 9, comprising a holding mechanism engageable to the guiding section for holding the surgical tool, the holding mechanism having:
a connecting member pivotably engaged to the guiding section; and
a holder detachably engageable to the connecting member, the holder configured to support the surgical tool.

11. The guiding system of claim 10, wherein the connecting member includes a longitudinal arm configured to be received between fingers of a user and a handle configured to be engaged by a hand of the user, the longitudinal arm protruding transversally from the handle, the connecting member having a support member secured to the handle, the holder detachably secured to the support member.

12. An ultrasonic transducer including the guiding system of any one of claims 1 to 11.

13. The ultrasonic transducer of claim 12, comprising a cover engaged to the body of ultrasonic transducer, the reference member releasably secured to the collar.

14. The ultrasonic transducer of claim 13, wherein the collar and the body of ultrasonic transducer are parts of a single monolithic body.

15. A method of guiding a surgical tool using an ultrasonic transducer, comprising:
positioning the ultrasonic transducer relative to a patient until a scanning plane of the ultrasonic transducer intersects a blood vessel of the patient;
magnetically connecting a guiding member to an attachment member, the attachment member being mounted to the ultrasonic transducer; and
engaging the surgical tool to the guiding member.
